# EUROPEAN PATENT APPLICATION

(11) **EP 3 248 602 A1**
(43) Date of publication of application: **29.11.2017**
(21) Application number: 16001199.5
(22) Date of filing: 26.05.2016
(51) Int. Cl.: A61K 31/502, A61P 19/02, A61K 45/00

(54) **USE OF 5-AMINO-2,3-DIHYDRO-1,4-PHTHALAZINEDIONE IN THE TREATMENT OF INFLAMMATORY AND/OR DEGENERATIVE DISORDERS OF THE TENDONS, LIGAMENTS OF THE JOINTS, ARTICULAR CAPSULES AND BURSAE**

(71) Applicant: MetrioPharm AG, 8002 Zürich (CH)
(72) Inventor: The designation of the inventor has not yet been filed

(57) **Abstract**

The use of 5-amino-2,3-dihydro-1,4-phthalazinedione, its complexes, solvates, hydrates, crystalline polymorphs, tautomers, isomers, isotopically enriched forms, prodrugs or their pharmaceutically acceptable salts in the prophylaxis and/or treatment of inflammatory and/or degenerative disorders of the tendons, ligaments of the joints, articular capsules and bursae is disclosed, as well as suitable application forms. This medical use is particularly beneficial in poorly perfused tissues.

## Description

Soft tissue is a generic term for tissues that connect, support or surround organs or other tissues and body structures. Soft tissue consists of a highly deformable cell matrix which at the same time confers flexibility and it is essential for keeping organs or body structures in their place inside the body. The term "soft tissue" refers to connective tissue, muscles, blood vessels, nerves, tendons, ligaments, fascia, skin, fibrous tissues, adipose tissue and synovial membranes. Bones, cartilage, glia, teeth, nails, hair, epithelial tissue and interior organs, however, do not belong to soft tissues. The prevailing cell types are fibroblasts and the related chondroblasts. Fibroblasts are responsible for the production of fibers and ground substance. Their extracellular matrix contains mainly collagen, elastin and ground substance. Elastin confers a certain stiffness to the tissue and is able to absorb a major part of strain energy applied on this body region. Collagen and its numerous subtypes are fibrous structural proteins of great tensile strength. It makes up for about 25% to 35% of the whole-body protein content, thus being the most abundant protein in mammals. It mainly supports tissues and cell structures from the outside, but can also be found intracellularly.

Like virtually all tissues, soft tissues can undergo inflammatory states and pathologic tissue degeneration. The treatment of such inflammatory or degenerative disorders, the average duration of treatment and the outcome of such a treatment depends, however, on the specific soft tissue species. Thus there is no standardized therapy for these disorders. When tendons, ligaments, articular capsules and bursae are affected, the healing process is often lengthy and not always a complete restitution can be attained. In numerous cases, a drug therapy only addresses some symptoms and the outcome is not much higher or quicker than a conservative non-pharmaceutical self-healing process. This particularly occurs in poorly perfused tissues. Therefore current treatment regimens are often regarded as unsatisfactory. There is a medical need for new pharmaceutical treatment methods for these disorders. Therefore it is the task of the present patent application to provide a suitable pharmaceutical drug to meet this medical need. It is a further task to provide suitable application and treatment forms as well as pharmaceutically acceptable dosage forms and ways of administration for such a pharmaceutical drug.

Surprisingly, it was found that a systemic administration of 5-amino-2,3-dihydro-1,4-phthalazinedione sodium salt showed an excellent effect in the treatment of inflammatory and/or degenerative diseases of tendons, ligaments, articular capsules and bursae, as will be specified in detail in the Examples.

5-amino-2,3-dihydro-1,4-phthalazinedione belongs to the pharmaceutical class of the phthalazinediones. Compounds of this class are known for their beneficial anti-inflammatory actions. 5-amino-2,3-dihydro-1,4-phthalazinedione is also known under the name luminol. Luminol became known for its chemiluminescent properties. It is widely applied in diagnostic assays as a detection means and in forensic medicine, for example for tracing blood spots. In medicine, 5-amino-2,3-dihydro-1,4-phthalazinedione has been developed in the form of a sodium salt. It is approved for a broad range of acute and chronic inflammatory disorders, including a.o. acute infections of bacterial and viral origin, particularly of the intestinal tract, hepatitis B and C, gastroenteritis, inflammations such as prostatitis, endometriosis, throat inflammation, bronchial asthma, pneumonia, periodontitis, pyelonephritis and autoimmune diseases such as Crohn's disease, ulcerative colitis, lupus erythematosus and scleroderma. Further, there is still a long list of indications in scientific and patent literature in the treatment of which 5-amino-2,3-dihydro-1,4-phthalazinedione was allegedly tested or a beneficial use was suggested (cf. WO 2004/041169; WO 2007/018546; WO 2012/127441 a.o.).

A use of 5-amino-2,3-dihydro-1,4-phthalazinedione or a related compound in the prophylaxis and/or treatment of inflammatory and/or degenerative diseases of tendons, ligaments, articular capsules and bursae, however, hasn't been described yet.

While most conventional immunomodulatory drugs have serious adverse effects, or are at least problematic in long-term treatment, 5-amino-2,3-dihydro-1,4-phthalazinedione or related compounds are well tolerated and have a high safety margin in respect to administered dosages.

To ensure a better solubility and bioavailability pharmaceutically acceptable salts of 5-amino-2,3-dihydro-1,4-phthalazinedione are used. Sodium, potassium and lithium salts have been described for therapeutic applications (cf. WO 2010/082858). Crystal structures for lithium, sodium, potassium, rubidium and cesium salts were described in Guzel et al. (2013) Journal of Coordination Chemistry 66, 3722-3739. Thus the present patent application refers also to the use of all pharmaceutically acceptable salts of 5-amino-2,3-dihydro-1,4-phthalazinedione and related compounds.

5-amino-2,3-dihydro-1,4-phthalazinedione is often used as a hydrate, for example as sodium salt dihydrate. Thus the present patent application refers also to the use of all hydrates and other solvates of 5-amino-2,3-dihydro-1,4-phthalazinedione and its pharmaceutically acceptable salts. 5-amino-2,3-dihydro-1,4-phthalazinedione, its derivatives or pharmaceutically acceptable salts may build complexes with suitable ligands. Thus the present patent application refers also to such complexes.

In order to ensure a reproducible and standardized API production and to provide improved stability features of an active agent anhydrous formulations are often preferred. Anhydrate forms of 5-amino-2,3-dihydro-1,4-phthalazinedione sodium salt have been described as crystalline polymorphs in WO 2011/107295 (Form I, Form II) and EP 14004274.8 (Form III). These crystalline polymorphs are virtually free from phase impurities and were characterized by means of X-ray powder diffraction. This method yields a set of characteristic d-values indicating interplanar spacings and of the corresponding 2-theta (2θ) angles under which Bragg reflections occur. Additionally, the relative intensities (upon normalization to the respectively highest peak as 100%) of the reflections are indicated therein. This yields a unique and unambiguous fingerprint of the respective polymorphs.

For Form I the following values were determined:
d values: 13.5; 6.9; 5.2; 4.6; 3.9; 3.5; 3.4; 3.3; 3.1; 3.0 and/or
   2-theta values: 6.5; 12.7; 16.9; 19.3; 22.8; 25.8; 26.6; 27.2; 28.7; 30.3.

Form II is characterized by the following values:
d values: 12.9; 7.9; 7.1; 6.5; 5.3; 4.0; 3.7; 3.6; 3.3; 3.2 and/or
   2-theta values: 6.8; 11.2; 12.5; 13.7; 16.7; 22.4; 24.3; 24.9; 27.2; 27.8.

Form III yielded the following values:
d values: 13.131; 7.987; 7.186; 6.566; 6.512; 5.372; 3.994; 3.662; 3.406; 3.288; 3.283; 3.222; 3.215; 3.127; 2.889 and/or
2-theta values: 6.73; 11.07; 12.31; 13.48; 13.59; 16.49; 22.24; 24.29; 26.14; 27.10; 27.14; 27.67; 27.72; 28.52; 30.93.

Thus the present patent application refers also to the use of all crystalline forms and polymorphs thereof of 5-amino-2,3-dihydro-1,4-phthalazinedione and its pharmaceutically acceptable salts.

Similar therapeutic effects are known for a variety of phthalazinediones, respectively of derivatives of 5-amino-2,3-dihydro-1,4-phthalazinedione and its pharmaceutically acceptable salts. An example is 6-amino-2,3-dihydrophthalazine-1,4-dione (isoluminol). An overview of suitable phthalazinediones is given in WO 2007/018546. It is reasonable to assume that these compounds show comparable effects when being used for the therapeutic applications according to the invention.

Tautomerism relates to a rapid intraconversion of organic compounds in which a hydrogen atom or proton formally migrates inside the compound. This is accompanied by a switch of a single bond and adjacent double bond. The single forms are called tautomers. For example, keto-enol tautomerism occurs in 5-amino-2,3-dihydro-1,4-phthalazinedione (Proescher and Moody, 1939, J Lab Clin Med, p. 1183-1189). Thus the present patent application refers also to the use of all tautomers of 5-amino-2,3-dihydro-1,4-phthalazinedione and its pharmaceutically acceptable salts.

Isomer is a generic term for molecules with the same chemical formula but a different chemical structure. They can be differentiated into constitutional (structural) isomers (wherein an exchange of atoms or of a functional group occurs) and stereoisomers. Stereoisomers can be subdivided into enantiomers (non-superimposable mirror images of the same molecule) and diastereomers (the same molecule with a different configuration at one or more stereocenters). Diastereomers can be subdivided into cis/trans isomers (referring to the relative orientation of functional groups within a molecule) and on the other hand conformers (rotation about formally single bonds) and rotamers (different rotational positioning about a single bond). An example for a constitutional isomer of 5-amino-2,3-dihydro-1,4-phthalazinedione is 6-amino-2,3-dihydrophthalazine-1,4-dione (isoluminol). Stereoisomers may occur in phthalazinedione derivatives. Thus the present patent application refers also to the use of all isomers of 5-amino-2,3-dihydro-1,4-phthalazinedione, its derivatives and pharmaceutically acceptable salts.

For some applications it may be desirable that isotopically enriched forms of the compounds of the invention are used, e.g. for diagnostic purposes. Thus the present patent application refers also to such isotopically enriched forms of the compounds of the invention.

From a pharmacokinetic point of view or for a production rationale it may be preferable to use a prodrug as a dosage form. A prodrug is administered in a pharmacologically inactive form and is metabolically converted into the active form inside the body. This conversion may occur systemically or locally. Thus the present patent application refers also to prodrugs of the compounds of the invention.

As used throughout the present application the term "5-amino-2,3-dihydro-1,4-phthalazinedione" shall encompass all the aforementioned molecular variants of 5-amino-2,3-dihydro-1,4-phthalazinedione, unless otherwise stated.

In the sense of the present patent application the term "inflammatory diseases" refers to diseases, disorders or other body conditions in which an inflammation becomes manifest as a major symptom. An inflammation is the response of body tissues to irritation (exogenous or endogenous noxae) or injury. It can be provoked amongst others by physical, chemical and biologic stimuli, comprising mechanical trauma, excessive sun exposure, radiation damage, corrosive chemicals, extremes of heat or cold, infectious agents such as bacteria, viruses, fungi and other pathogenic microorganisms or parts of them. An inflammation can have beneficial and/or detrimental effects in the affected tissue(s). At a first stage an inflammation is regarded as acute. When it isn't terminated after some time the inflammation may become chronic. Typical signs of an inflammation are redness, swelling, heat development, pain and reduced functionality. This may even lead to a loss of function of the affected tissue.

An inflammation is one of the first responses of the immune system that has become activated e.g. by an infection or degenerated endogenous cells. The system of innate immunity mediates an unspecific response, amongst others a general inflammatory response, while the adaptive immune system provides reactions specific to the respective pathogen, which will then be remembered by the immune system. An organism can be in an immunodeficient state, i.e. the immune response is not able to cope with the aforementioned irritations or injuries in a satisfactory manner. On the other hand the immune system might become hyperactive and turn its defense against endogenous tissues, as in the case of autoimmune diseases. Thus the present patent application refers also to a use in inflammatory diseases which come into being as a sequela of immunodeficiency, respectively to a use in pharmacologically induced immunosuppression, as well to a use in disease states due to a respective autoimmune disease.

In the sense of the present patent application the term "degenerative diseases" refers to diseases, disorders or other body conditions in which a continuous process leads to degenerative cell changes. The affected tissues or organs deteriorate continuously over time. Such a degeneration may be due to physical or physiological over-exercise of specific vulnerable body structures, life style, eating habits, age, congenital diseases or other endogenous causes. The degeneration can be caused or accompanied by an atrophy or dystrophy of the respective tissue or organ. Often a loss of function and/or an irreversible damage of the affected tissue or organ occurs.

In the sense of the present patent application the terms "lesion", "microlesion" and "trauma" refer to injuries of different size and scope in the affected soft tissue. They can be inflicted by spontaneous physical impact in which the impacting force or torque leads to a tissue damage. But they can also be the final consequence of a previous degenerative disease of the affected soft tissue, or vice versa a microlesion may be the starting point of such a degenerative disease in the wake of the microlesion. Also an inflammation of the affected soft tissue can favor such a microlesion or trauma, or it can be their sequelae. So these terms are interconnected with inflammation and degenerative disease. Therefore the aspects of lesion, microlesion and trauma of the affected soft tissues that don't refer to wound healing in the narrow sense may be subsumed under inflammatory and/or degenerative diseases of said tissues.

If it is known that a healthy person is or will be vulnerable to inflammatory or degenerative diseases, or a tissue damage is to be expected due to a constant overstrain of the respective tissue or organ it can be indicated to give a prophylactic medication in order to prevent or at least to mitigate the expected impairment or damage. Thus the present patent application refers also to a prophylactic use according to the invention.

There are also cases in which an inflammatory disease results in a degenerative disease. Examples therefore will be given further below. Thus the present patent application refers to the use according to the invention in the prophylaxis and/or treatment of inflammatory and/or degenerative diseases.

The anatomic term soft tissue relates to tissues that connect, support or surround other structures and organs of the body. Bone tissue is not included. Such soft tissues include connective tissue, tendons, ligaments, fascia, skin, fibrous tissue, adipose tissue, synovial membranes, but also muscles, nerves and blood vessels. In histology, soft tissue is defined as non-epithelial, extraskeletal mesenchyme exclusive of the reticuloendothelial system and glia (Skinner (2006): Current diagnosis & treatment in orthopedics. Stamford, Conn, USA. Lange Medical Books/McGraw Hill, p. 346). As soft tissues include very different tissues in respect of their histological structure and their physiological functionality, the manifestation of inflammatory and/or degenerative diseases of these tissues, their severity and incidence is also highly variable, and thus is their treatment.

For example, muscles, nerves and blood vessels have a different structure and physiology than collagen-rich soft tissues like tendons, ligaments, articular capsules and bursae.

### Tendons

A tendon (synonym: sinew) is a fibrous viscoelastic band of connective tissue that connects muscle and bone. Due to its specific structure it can withstand expansion stress. It serves for force transmission for moving a bone, depending on the contraction or relaxation of the respective skeletal muscle.

The entire tendon structure is built by the tendon itself in the center, delimited by the synovial covering. This structure is surrounded by the tendon sheath, delimited to the outside by the fibrous sheath and to the inside by the synovial lining of the tunnel. The tunnel or synovial cavity is a cavity inside the tendon sheath around the tendon and is filled with synovial fluid. The mesotendon builds a small connection between the tendon and the tendon sheath. Thus the tendon is capable to glide longitudinally inside the tendon sheath, the friction being reduced by the synovial fluid. The synovial fluid serves also for shock absorption and provision of nutrients to the tendon. As lubricating components for increasing the viscosity of the synovial fluid serve hyaluronic acid and lubricin (PRG4). There is only a poor innervation and blood supply of the inner tendon. In the sense of the present application the terms tendon and inflammatory and/or degenerative disorders of tendons shall relate to the entire tendon structure, respectively to all parts of the entire tendon structure, as described above.

Tendons are composed of closely packed parallel arrays of collagen fibers. Their total dry mass consists of ca. 86% collagen, 2% elastin, 1-5% proteoglycans and 0.2% inorganic components (Lin et al. (2003), J Biomechanics 37, p. 865-877).

Collagen is one of the main structural proteins in metazoa. It is present inside cells, but it is also attached to the cell membrane on the outside via other proteins. In humans collagen accounts for over 30% of the overall protein content. Collagen is a single long-chained protein forming a left-handed helix. Three of these polypeptide chains are intertwined in the form of a right-handed helix. 28 collagen subtypes are known until now among which subtypes I to V are the most common. The abundant collagen subtype in healthy tendon tissue is collagen I.

The term tendinitis relates mainly to an acute inflammation of a tendon. A tendinitis may be idiopathic, but is often due to a tendon injury. Symptoms can include pain, local joint stiffness, a burning sensation, and swelling with heat and redness. Sometimes visible knots around the affected joint develop. Pain usually worsens during and after straining of the affected joint. A tendinitis can materialize in all tendons, it is particularly common in the upper shoulder and the lower section of the elbow.

### Tendinitis

Conventional tendinitis, respectively tendovaginitis, occurs often after over-exercise of the elbow ("tennis elbow"), the wrist ("mouse hand") or the big toe, respectively the metatarsophalangeal joint due to long walks. The treatment is mainly conservative non-pharmaceutical and includes resting. Rest shall prevent further damage of the affected tendon. Gradual return to exercise ensues. Cooling with ice, compression or elevation of the affected limb may mitigate acute symptoms. Pharmaceutical treatment consists above all in the use of NSAIDs (non-steroidal anti-inflammatory drugs), aiming at the blockade of cyclooxygenase (COX-1 and/or COX-2) and thus the production of thromboxanes and prostaglandins. Systemic administration of steroids such as cortisone and dexamethasone shows only good short-term effects. The therapy with NSAIDS or steroids causes, however, serious adverse effects. The long-term use of NSAIDs may lead to gastrointestinal problems including dyspepsia and bleeding; kidney problems such as salt and fluid retention, hypertension, renal failure, and interstitial nephritis; cardiovascular problems such as an increased risk for heart failure, myocardial infarction, and stroke; several undesired drug interactions, and possibly erectile dysfunction. Another problem in the use of NSAIDs in the diseases or states of the invention is that they rather mask the pain but don't treat the underlying inflammation or degenerative disease. Therefore patients are often tempted to overstress the muscle belonging to the affected tendon, as they don't feel the pain anymore. By such a behavior they might even worse the inflammation or degenerative disease. From this point of view the use of NSAIDs herein might be even contraindicated. Continuous use of cortisone may lead amongst others to Cushing's syndrome, osteoporosis, hyperglycemia, diabetes mellitus, amenorrhea, anxiety, depression, cataracts, and glaucoma; dexamethasone treatment may cause amongst others to weight gain, impaired skin healing, hypertension, nausea, dyspepsia, headache, cataracts, insomnia, and depression.

Thus neither the use of NSAIDs nor of the mentioned steroids is uncomplicated. Moreover, in many cases the beneficial outcome of such a treatment is poor, particularly in the light of the risk of the described side effects. Therefore a conservative non-pharmaceutical but lengthy treatment is often preferred. Thus there is a medical need for an improved pharmaceutical therapy of tendinitis, or at least for an alternative to the conventional pharmaceutical treatment options. A higher efficiency or at least less adverse effects are desirable. Thus it was the task of the present patent application to provide such a novel pharmaceutical treatment option.

The term tendinosis should be differentiated from tendinitis. Tendinosis starts when the acute inflammation (tendinitis) turns into a chronic tendon injury. This phase starts when the acute inflammation is about to subside. Thus often a continuous transition from tendinitis to tendinosis in a patient's tendon can be observed. This phase can be described as a chronic degenerative tendopathy without inflammation. It is assumed that it is caused by microtears, respectively microlesions in the connective tissue of the tendon. The degeneration affects mostly the collagenous matrix and includes hypercellularity, hypervascularity and a lack of inflammatory cells.

As is specified in more detail in Examples 1, 2 and 4, the systemic administration of 5-amino-2,3-dihydro-1,4-phthalazinedione sodium salt surprisingly leads to a significantly quicker healing and even restitution of the degenerated tissue, with a simultaneous disappearance of the symptoms and a restitution of tendon functionality, in comparison to a conservative physiotherapeutical or a conventional pharmaceutical therapy.

Thus the use of the compounds according to the invention refers also to the treatment of tendinosis, or a combined tendinitis / tendinosis.

### Ligaments

In vertebrates including humans, the term ligament relates to fibrous connective tissue that connects bones to other bones, allowing them to move inside a joint and at the same time limiting the flexibility of the joints to a physiologically reasonable degree. The present invention does not relate to outspread ligaments such as the peritoneal ligament, or for example the periodontal ligaments. Their morphology, tethering, function and pathology differs from the fibrous bone-to-bone ligaments. Therefore the terms ligament(s) and ligaments of the joints shall be used throughout the description, referring to bone-to-bone ligaments only.

Also ligaments are viscoelastic. Under tension they are extended and will return to their resting shape upon removal of the strain stress. They consist mainly of collagen (ca. 75% of total dry mass, with collagen I amounting for ca. 85% of overall collagen), elastin (to a higher content than in tendons), lipids, proteoglycans, glycoproteins and optionally an epiligament coat (cf. Frank (2004), J Musculoskel Neuron Interact 4, p. 199 - 201).

In comparison to tendons ligaments are more often torn in traumatic joint injuries which may lead to a partial or complete ligament disconnection. Also an overstretched ligament is a common disorder. They are also referred to as sprains. But also inflammatory and/or degenerative diseases occur in ligaments. In general, ligament disorders are classified into three phases: a) hemorrhage with inflammation, b) matrix and cellular proliferation, c) remodeling and maturation (cf. Frank (2004), J Musculoskel Neuron Interact 4, p. 199 - 201). Most sprains or ligament lesions are treated conservatively with immobilization of the affected joint, cooling, compresses against the swelling and elevation of the affected joint. In cases of a ligament rupture a surgery may be necessary. Artificial ligaments, for example made polyNaSS (poly sodium p-styrenesulfonate), can be used as endoprosthesis. There is no established pharmaceutical treatment, unless a symptomatic treatment with analgesics and/or anticoagulants. Glucocorticoids are only reluctantly administered because of a poor risk-benefit ratio.

### Articular capsules

Articular capsules (synonym: joint capsules) are envelopes that surround a synovial joint. They consist of two layers, an outer fibrous layer (fibrous membrane; fibrous stratum), and an inner synovial layer (synovial membrane, synovial stratum). The outer layer consists mainly of avascular white fibrous tissue, while the inner layer is a secreting layer. Where the inside of the capsules gets into contact with the outer layers of the bones, articular cartilage covers the end surfaces of the bones articulating in this joint. The outer layer is densely innervated with nociceptors and hosts many proprioceptors. Therefore it is particularly vulnerable to inflammatory pain. It is attached to the whole circumference of the articular end of each bone and thus surrounds the articulation entirely. It consists of dense irregular connective tissue. Collagen I is the abundant component of this connective tissue.

Common inflammations of the articular capsule include frozen shoulder (adhesive capsulitis) and the plica syndrome. These inflammations are often treated with NSAIDs or corticosteroids sometimes in combination with exercise of the affected joint, or iontophoresis, phonophoresis or laser techniques. However, NSAID and corticosteroid treatments have the known drawbacks.

### Bursae

A bursa is a small fluid-filled sac lined by the synovial membrane. It serves like a cushion between the bones and tendons and muscles around a joint. By this way the friction between the bones is diminished and a free articulation is enabled. They are filled with synovial fluid. As articular capsules and tendon sheaths they consist of an outer stratum fibrosum and an inner stratum synoviale. This stratum synoviale is an inner capillary layer that excretes a viscous fluid.

Infection or irritation of a bursa leads to a bursitis, the most common form of a bursopathy. Also in the bursae the stratum fibrosum consists abundantly of collagen I. A coating of synovial cells with collagen and proteins may be affected by the inflammatory process.

Also bursitis is commonly treated with NSAIDs and corticosteroids, in case of an underlying bacterial infection also with antibiotics. Careful physiotherapy may alleviate the symptoms.

Thus the present patent application refers to 5-amino-2,3-dihydro-1,4-phthalazinedione, its complexes, solvates, hydrates, crystalline polymorphs, tautomers, isomers, isotopically enriched forms, prodrugs or their pharmaceutically acceptable salts, for use in the prophylaxis and/or treatment of inflammatory and/or degenerative disorders of the tendons, ligaments of the joints, articular capsules and bursae.

The common denominator of the inflammatory and/or degenerative disorders of the tendons, ligaments of the joints, articular capsules and bursae is apparently the collagen-containing tissue layers, namely the high content of collagen I. From pathophysiology it is known that in cases of injuries (trauma) or microlesions of such collagen-containing soft tissues the repair starts with an over-expression of extracellular matrix proteins such as tenascin C and fibronectin, ensued by an over-expression of collagen III which takes the place of damaged collagen I in the cellular scaffold.

Collagen III, however, has less favorable viscoelastic properties than collagen I. Therefore tendons, for example, are less flexible and resilient during the healing process. On the other hand, there is an increased strain plasticity that endows the damaged tissue with a high retraction capacity. This also helps to protect the local blood vessels and it allows a differential activation of fiber bundles during the healing process. This helps to minimize the scarring, respectively necrosis of the damaged tissue. Thus in a first phase of soft tissue repair the affected joint or muscle is less flexible, respectively resilient than during its unaffected state. This is also a common experience by patients affected by such an injury. In the course of time (namely the ensuing two or three weeks) collagen III is going to be continuously replaced by collagen I until the healthy state and the favorable properties of tendon function are completely reconstituted. Finally, ca. 90% of total collagen will be collagen I and ca. 10 % collagen III.

An explanation might be that in the event of a minor or major lesion of the aforementioned soft tissues 5-amino-2,3-dihydro-1,4-phthalazinedione influences the expression pattern of the involved structural proteins during the healing process in a positive way. Thus the healing is supported and accelerated so that a substantially complete restitution of the affected tissue is achieved in a shorter time.

Therefore it can also be said that the present patent application refers to the prophylaxis and/or treatment of inflammatory and/or degenerative disorders of collagen-rich soft tissues. Preferred is the prophylaxis and/or treatment of inflammatory and/or degenerative disorders of soft tissues physiologically containing a high percentage of collagen I.

As tendons, ligaments, articular capsules and bursae can be subsumed as soft tissues of the joints it can be said alternatively that the present patent application refers also to the prophylaxis and/or treatment of inflammatory and/or degenerative disorders of soft tissues of the joints, respectively of soft tissues, wherein the soft tissues are selected from the group comprising tendons, ligaments of the joints, articular capsules and bursae.

One major problem of the pharmaceutical treatment in the prophylaxis and/or treatment of the diseases addressed herein is that the described soft tissues are often poorly perfused. Thus only a minor percentage of a systemically administered drug actually reaches the diseased tissue and therefore the efficacy is less than satisfactory. Alternatively, the dosage of the systemically administered drug must be increased considerably to achieve the desired drug effects. But this means at the same time that unwanted side effects of these drugs, namely of NSAIDs and corticosteroids, become much more manifest which severely hampers the use or at least the long-term use of these drugs in high concentrations. Therefore, there is a medical need to find drugs that don't show these difficulties. Herein the use of 5-amino-2,3-dihydro-1,4-phthalazinedione sodium salt is advantageous, as this drug provides an excellent safety profile and is very well tolerated. Therefore this drug is virtually predestined for systemic administration in diseases that become manifest in rather poorly perfused tissues, as in the present case. The safety profile allows also higher than usually needed doses to be administered in the treatment of said soft tissue diseases. This ensures that a sufficiently high dose reaches said poorly perfused tissues. Therefore the present patent application refers also to the use of the compounds of the invention the prophylaxis and/or treatment of inflammatory and/or degenerative disorders of the tendons, ligaments of the joints, articular capsules and bursae that become manifest in poorly perfused tissues.

The perfusion rate of tissues correlates with the oxygen saturation. Oxygen saturation can be monitored for example by means of pulse oximeters known in the art. Normal blood oxygen levels in humans, respectively endotherm animals, are considered to be between 95% and 100%. A 100% oxygen saturation corresponds roughly to oxygen partial pressures of > 10 kPa. Levels below 90% are regarded as low, resulting in hypoxemia. Therefore the present patent application refers also to the use of the compounds of the invention the prophylaxis and/or treatment of inflammatory and/or degenerative disorders of the tendons, ligaments of the joints, articular capsules and bursae that become manifest in poorly perfused tissues in which the poor perfusion correlates with an oxygen saturation below 90%, preferred below 85%, more preferred below 80%, more preferred below 75%, still more preferred below 70%, even more preferred below 65% and most preferred below 60%.

The compound according to the invention can be used in the prophylaxis and/or treatment of inflammatory and/or degenerative disorders of any tendon. These disorders can be selected from a group, without being limiting, that comprises tendinopathy; tendopathy; tendinitis; tenosynovitis; infective tenosynovitis; tendinosis; abscess of tendon sheath; calcific tendinitis; trigger finger; radial styloid tenosynovitis; tendovaginitis stenosans; contracture of the tendon or the tendon sheath; tenosynovitis as a symptom of gonorrhea, syphilis or tuberculosis; chronic tenosynovitis of hand and wrist with or without crepitus; tendinitis trochanterica; rotator cuff tendinopathy; inflammations, lesions or tears of the rotator cuff tendons; bicipital tendinitis; calcific tendinitis of shoulder; supraspinatus syndrome; gluteal tendinitis; psoas tendinitis; enthesopathy; iliotibial band syndrome; patellar tendinitis; Achilles tendinopathy; Achilles tendinitis; peroneal tendinitis; tibialis anterior tendinitis; tibialis posterior tendinitis; tibialis posterior tendinitis; posterior tibial tendon dysfunction (PTTD); medial epicondylitis; lateral epicondylitis and repetitive strain injury syndrome (RSI) as e.g. tennis elbow or mouse arm.

The compound according to the invention can be used in the prophylaxis and/or treatment of inflammatory and/or degenerative disorders of any ligament of the joints. These disorders can be selected from a group, without being limiting, that comprise ligamentopathy; ligamentitis; stenosing ligamentitis; sacroiliitis; irritations, stretches and tears of the ligaments; and ligamentous laxity.

The compound according to the invention can be used in the prophylaxis and/or treatment of inflammatory and/or degenerative disorders of any articular capsule. These disorders can be selected from a group, without being limiting, that comprise synoviopathy; synovitis; infective synovitis; rupture of synovium; synovial hypertrophy; transient synovitis; synovial cysts; Baker's cyst; capsulitis; adhesive capsulitis of the shoulder; adhesive capsulitis of the hip and synovitis as a symptom of gonorrhea, syphilis or tuberculosis as well as of an autoimmune disease.

The compound according to the invention can be used in the prophylaxis and/or treatment of inflammatory and/or degenerative disorders of any bursa. These disorders can be selected from a group, without being limiting, that comprise bursopathy; bursitis; infective bursitis; bursitis of hand; Olecranon bursitis; bursitis of elbow; prepatellar bursitis; infrapatellar bursitis; bursitis of knee; trochanteric bursitis; bursitis of hip; subacromial bursitis; Achilles bursitis; retrocalcaneal bursitis; ischial bursitis; iliopsoas bursitis; anserine bursitis; abscess of bursa; bursal cyst; bursitis of shoulder; calcific bursitis; and bursitis as a symptom of gonorrhea, syphilis or tuberculosis as well as of an autoimmune disease.

The present application refers likewise to a composition for use in the prophylaxis and/or treatment of inflammatory and/or degenerative disorders of the tendons, ligaments of the joints, articular capsules and bursae, wherein said composition contains at least one of 5-amino-2,3-dihydro-1,4-phthalazinedione or any of aforementioned molecular variants of this compound, a carrier and at least one excipient selected from a group comprising penetration enhancers; binding agents; solvents; solubilizing agents; buffers; preservatives; antioxidants; coatings; colorants; flavoring substances; aromatic substances; sweeteners; thickening agents; disintegrants; glidants; lubricants; emulsifiers; stabilizers; diluents; anti-caking agents (antiadherents); sorbents and opacifiers.

Eligible carriers are all carriers known in the art and combinations thereof. In solid dosage forms they can be for example plant and animal fats, waxes, paraffins, starch, tragacanth, cellulose derivatives, polyethylene glycols, silicones, bentonites, silica, talcum, zinc oxide. For liquid dosage forms and emulsions suitable carriers are for example solvents, solubilizing agents, emulsifiers such as water, ethanol, isopropanol, ethyl carbonate, ethyl acetate, benzyl alcohol, benzyl benzoate, propylene glycol, 1,3-butyl glycol, cotton seed oil, peanut oil, olive oil, castor oil, sesame oil, glycerol fatty acid esters, polyethyl glycols, fatty acid esters of sorbitan. Suspensions may use carriers known in the art such as diluents (e.g. water, ethanol or propylene glycol), ethoxylized isostearyl alcohols, polyoxyethylene and polyoxyethylene sorbitan esters, microcrystalline cellulose, bentonites, agar agar, tragacanth. Permeation enhancers are often used in topical dosage forms. Suitable permeation enhancers comprise all pharmaceutically acceptable permeation enhancers known in the art, such as, without being limiting, azones such as laurocapran, 1-dodecylazacycloheptan-2-one; sulphoxides such as dimethylsulphoxide, DMAC, DMF; pyrrolidones such as 2-pyrrolidone, N-methyl-2-pyrrolidone; alcohols such as ethanol, 1,2-propandiol or decanol; glycols such as propylene glycol, diethylene glycol, tetraethylene glycol; fatty acids such as oleic acid, lauric acid, sodium lauryl sulfate, myristic acid, isopropyl myristic acid, capric acid; nonic surfactants such as polyoxyethylene-2-oleyl ether, polyoxyethylene-2-stearyl ether; terpenes; terpenoids; oxazolidinones; urea; ceramide analogs, azone analogs, menthol derivatives, etherified derivatives, esterified derivatives, transkarbams, carbamate salts, TXA derivatives, DDAIP (dodecyl 2-(dimethylamino) propanoate), DDAK, natural essential oils (all of them listed in Chen et al. (2014) Asian J. Pharm. Sc. 9, 51-64); citric acid esters such as triethyl citrate; hydrophobin polypeptides; alpha-bisabolol; dimethyl isosorbide (Arlasove^{®} DMI); ethoxydiglycol. Preferred is 1,2-propandiol.

The term binding agents refers to substances that bind powders or glue them together, rendering them cohesive through granule formation. They serve as a "glue" of the formulation. Binding agents increase the cohesive strength of the provided diluent or filler.

Suitable binding agents are starch from wheat, corn, rice or potato, gelatine, naturally occurring sugars such as glucose, sucrose or beta-lactose, sweeteners from corn, natural and synthetic gums such as acacia, tragacanth or ammonium calcium alginate, sodium aliginate, carboxymethyl cellulose, sodium carboxymethyl cellulose, hydroxypropyl carboxymethyl cellulose, polyethylene glycol, polyvinyl pyrrolidone, magnesium aluminium sillicate, waxes and others. The percentage of the binding agent in the composition can range from 1 - 30 % by weight, preferred 2 - 20 % by weight, more preferred 3 - 10 % by weight and most preferred 3 - 6 % by weight.

Suitable solvents may be selected from the group comprising water, carbonated water, water for injection, water with isotonizing agents, saline, isotonic saline, alcohols, particularly ethyl and n-butyl alcohol, glycols, oleic and linoleic acid triglycerides, caprylic and capric acid mono-, di- and triglycerides, polyoxyethylene caprylic and capric acid glycerides, propylene glycol fatty acid esters, low alkyl fatty acid esters, soy bean oil, propylene glycol laurate, polyoxyethylene (35) castor oil, polyoxyethylene glyceryl trioleate, ethyl butyrate, ethyl caprylate, ethyl oleate and mixtures thereof.

Suitable as surface-active solubilizing agents (solubilizers) are for example diethylene glycol monoethyl ester, polyethyl propylene glycol co-polymers, cyclodextrins such as α- and β-cyclodextrin, glyceryl monostearates such as Solutol HS 15 (Macrogol-15-hydroxystearate from BASF, PEG 660-15 hydroxystearates), sorbitan esters, polyoxyethylene glycol, polyoxyethylene sorbitanic acid esters, polyoxyethylene sorbitan monoleate, polyoxyethylene oxystearic acid triglyceride, polyvinyl alcohol, sodium dodecyl sulfate, (anionic) glyceryl monooleates etc.

Moreover, buffers or buffer solutions are preferred for liquid formulations, in particular for pharmaceutical liquid formulations. The terms buffer, buffer system and buffer solution, in particular of an aqueous solution, refer to the capacity of the system to resist a pH change by the addition of an acid or a base, or by dilution with a solvent. Preferred buffer systems may be selected from the group comprising formate, lactate, benzoic acid, oxalate, fumarate, aniline, acetate buffer, citrate buffer, glutamate buffer, phosphate buffer, succinate, pyridine, phthalate, histidine, MES (2-(N-morpholino) ethanesulfonic acid, maleic acid, cacodylate (dimethyl arsenate), carbonic acid, ADA (N-(2-acetamido)imino diacetic acid, PIPES (4-piperazine-bis-ethanesulfonic acid), BIS-TRIS propane (1,3-bis[tris(hydroxymethyl)mehylaminol] propane), ethylene diamine, ACES (2-[(amino-2-oxoethyl)amino]ethanesulfonic acid), imidazol, MOPS (3-(N-morphino)-propanesulfonic acid, diethyl malonic acid, TES (2-[tris(hydroxymethyl)methyl]aminoethanesulfonic acid, HEPES (N-2-hydroxyethylpiperazine-N'-2-ethanesulfonic acid), as well as other buffers with a pKₐ between 3.8 and 7.7.

Preferred are carbonic acid buffers such as acetate buffer and dicarboxylic acid buffers such as fumarate, tartrate and phthalate as well as tricarboxylic acid buffers such as citrate.

A further group of preferred buffers are inorganic buffers such as sulfate hydroxide, borate hydroxide, carbonate hydroxide, oxalate hydroxide, calcium hydroxide and phosphate buffers. Another group of preferred buffers are nitrogen-containing puffers such as imidazol, diethylene diamine and piperazine. Furthermore preferred are sulfonic acid buffers such as TES, HEPES, ACES, PIPES, [(2-hydroxy-1,1-bis-(hydroxymethyl)ethyl)amino]-1-propanesulfonic acid (TAPS), 4-(2-hydroxyethyl)piperazine-1-propanesulfonic acid (EEPS), 4-morpholino-propanesulfonic acid (MOPS) and N,N-bis-(2-hydroxyethyl)-2-aminoethanesulfonic acid (BES). Another group of preferred buffers are glycine, glycyl-glycine, glycyl-glycyl-glycine, N,N-bis-(2-hydroxyethyl)glycine and N-[2-hydroxy-1,1-bis(hydroxymethyl)ethyl]glycine (tricine). Preferred are also amino acid buffers such as glycine, alanine, valine, leucine, isoleucine, serine, threonine, phenylalanine, tyrosine, tryptophan, lysine, arginine, histidine, aspartate, glutamate, asparagine, glutamine, cysteine, methionine, proline, 4-hydroxy proline, N,N,N-trimethyllysine, 3-methyl histidine, 5-hydroxy-lysine, o-phosphoserine, gamma-carboxyglutamate, [epsilon]-N-acetyl lysine, [omega]-N-methyl arginine, citrulline, ornithine and their derivatives.

Preservatives for liquid dosage forms or supplements can be used on demand. They may be selected from the group comprising sorbic acid, potassium sorbate, sodium sorbate, calcium sorbate, methyl paraben, ethyl paraben, methyl ethyl paraben, propyl paraben, benzoic acid, sodium benzoate, potassium benzoate, calcium benzoate, heptyl p-hydroxybenzoate, sodium methyl para-hydroxybenzoate, sodium ethyl para-hydroxybenzoate, sodium propyl para-hydroxybenzoate, benzyl alcohol, benzalkonium chloride, phenylethyl alcohols, cresols, cetylpyridinium chloride, chlorobutanol, thiomersal (sodium 2-(ethylmercurithio) benzoic acid), sulfur dioxide, sodium sulphite, sodium bisulphite, sodium metabisulphite, potassium metabisulphite, potassium sulphite, calcium sulphite, calcium hydrogen sulphite, potassium hydrogen sulphite, biphenyl, orthophenyl phenol, sodium orthophenyl phenol, thiabendazole, nisin, natamycin, formic acid, sodium formate, calcium formate, hexamine, formaldehyde, dimethyl dicarbonate, potassium nitrite, sodium nitrite, sodium nitrate, potassium nitrate, acetic acid, potassium acetate, sodium acetate, sodium diacetate, calcium acetate, ammonium acetate, dehydroacetic acid, sodium dehydroacetate, lactic acid, propionic acid, sodium propionate, calcium propionate, potassium propionate, boric acid, sodium tetraborate, carbon dioxide, malic acid, fumaric acid, lysozyme, copper-(II)-sulfate, chlorine, chlorine dioxide and other suitable substances or compositions known to the person skilled in the art.

The addition of antioxidants is particularly preferable in topical dosage forms. Suitable examples for antioxidants include sodium metabisulfite, alpha-tocopherol, ascorbic acid, maleic acid, sodium ascorbate, ascorbyl palmitate, butylated hydroxyanisol, butylated hydroxytoluol, fumaric acid or propyl gallate. Preferred is the use of sodium metabisulfite.

Tablets or pills are usually coated, i.e. the coating constitutes the outer layer. This can be a film coating, a sugar coating with saccharides and a compression coating. Pharmaceutically acceptable varnishes or waxes, HPMC, MC or HPC can be used. Such a coating may help to disguise the taste, to ease the swallowing or the identification. Often plasticizers and pigments are included in the coating.

Capsules normally have a gelatinous envelope that encloses the active substance. The specific composition and thickness of this gelatinous layer determines how fast absorption takes place after ingestion of the capsule. Of special interest are sustained release formulations, as known in the art.

Colorants are excipients that bestow a colorization to the composition of the drink, respectively the dosage form. These excipients can be food colorants. They can be adsorbed on a suitable adsorption means such as clay or aluminium oxide. The amount of the colorant may vary between 0.01 and 10 % per weight of the composition, preferred between 0.05 and 6 % per weight, more preferred between 0.1 and 4 % per weight, most preferred between 0.1 and 1 % per weight.

Suitable food colorants are curcumin, riboflavin, riboflavin-5'-phosphate, tartrazine, alkanin, quinolione yellow WS, Fast Yellow AB, riboflavin-5'-sodium phosphate, yellow 2G, Sunset yellow FCF, orange GGN, cochineal, carminic acid, citrus red 2, carmoisine, amaranth, Ponceau 4R, Ponceau SX, Ponceau 6R, erythrosine, red 2G, Allura red AC, Indathrene blue RS, Patent blue V, indigo carmine, Brilliant blue FCF, chlorophylls and chlorophyllins, copper complexes of chlorophylls and chlorophyllins, Green S, Fast Green FCF, Plain caramel, Caustic sulphite caramel, ammonia caramel, sulphite ammonia caramel, Black PN, Carbon black, vegetable carbon, Brown FK, Brown HT, alpha-carotene, beta-carotene, gamma-carotene, annatto, bixin, norbixin, paprika oleoresin, capsanthin, capsorubin, lycopene, beta-apo-8'-carotenal, ethyl ester of beta-apo-8'-carotenic acid, flavoxanthin, lutein, cryptoxanthin, rubixanthin, violaxanthin, rhodoxanthin, canthaxanthin, zeaxanthin, citranaxanthin, astaxanthin, betanin, anthocyanins, saffron, calcium carbonate, titanium dioxide, iron oxides, iron hydroxides, aluminium, silver, gold, pigment rubine, tannin, orcein, ferrous gluconate, ferrous lactate.

Suitable aromatic and flavoring substances comprise above all essential oil that can be used for this purpose. In general, this term refers to volatile extracts from plants or parts of plants with the respective characteristic smell. They can be extracted from plants or parts of plants by steam distillation.

Examples are: Essential oils, respectively aromatic substances from sage, cloves, chamomile, anise, star anise, thyme, tea tree, peppermint, mint oil, menthol, cineol, eucalyptus oil, mango, figs, lavender oil, chamomile blossoms, pine needles, cypress, oranges, rosewood, plum, currant, cherry, birch leaves, cinnamon, limes, grapefruit, tangerine, juniper, valerian, lemon balm, lemon grass, palmarosa, cranberry, pomegranate, rosemary, ginger, pineapple, guava, echinacea, ivy leave extract, blueberry, kaki, melons etc. or mixtures thereof, as well as mixtures of menthol, peppermint and star anise oil or menthol and cherry flavor.

These aromatic or flavoring substances can be included in the range of 0.0001 to 10 % per weight (particularly in a composition), preferred 0.001 to 6% per weight, more preferred 0.001 to 4% per weight, most preferred 0.01 to 1% per weight, with regard to the total composition. Application- or single case-related it may be advantageous to use differing quantities.

Suitable sweeteners can be selected from the group comprising mannitol, glycerol, acesulfame potassium, aspartame, cyclamate, isomalt, isomaltitol, saccharin and its sodium, potassium and calcium salts, sucralose, alitame, thaumatin, glycyrrhizin, neohesperidine dihydrochalcone, steviol glycosides, neotame, aspartame-acesulfame salt, maltitol, maltitol syrup, lactitol, xylitol, erythritol.

Suitable thickening agents can be selected from the group comprising polyvinyl pyrrolidone, methyl cellulose, hydroxypropyl methyl cellulose, hydroxypropyl cellulose, dextrins, polydextrose, modified starch, alkaline modified starch, bleached starch, oxidized starch, enzyme-treated starch, monostarch phosphate, distarch phosphate esterified with sodium trimetaphosphate or phosphorus oxychloride, phosphate distarch phosphate, acetylated distarch phosphate, starch acetate esterified with acetic anhydride, starch acetate esterified with vinyl acetate, acetylated distarch adipate, acetylated distarch glycerol, distarch glycerin, hydroxy propyl starch, hydroxy propyl distarch glycerine, hydroxy propyl distarch phosphate, hydroxy propyl distarch glycerol, starch sodium octenyl succinate, acetylated oxidized starch, hydroxyethyl cellulose.

Suitable disintegrants can be selected from the group comprising starch, cold water-soluble starches such as carboxymethyl starch, cellulose derivatives such as methyl cellulose and sodium carboxymethyl cellulose, microcrystalline cellulose and cross-linked microcrystalline celluloses such as croscarmellose sodium, natural and synthetic gums such as guar, agar, karaya (Indian tragacanth), locust bean gum, tragacanth, clays such as bentonite, xanthan gum, alginates such as alginic acid and sodium alginate, foaming compositions a.o. Moisture expansion is supported by for example starch, cellulose derivatives, alginates, polysaccharides, dextrans, cross-linked polyvinyl pyrrolidone. The amount of the disintegrant in the composition may vary between 1 and 40 % per weight, preferred between 3 and 20% per weight, most preferred between 5 and 10 % per weight.

Glidants are materials that prevent a baking of the respective supplements and improve the flow characteristics of granulations so that the flow is smooth and constant.

Suitable glidants comprise silicon dioxide, magnesium stearate, sodium stearate, starch and talcum. The amount of the glidant in the composition may vary between 0.01 and 10 % per weight, preferred between 0.1 and 7 % per weight, more preferred between 0.2 and 5 % per weight, most preferred between 0.5 and 2 % per weight.

The term lubricants refers to substances that are added to the dosage form in order to facilitate tablets, granulates etc. to be released from the press mold or the outlet nozzle. They diminish friction or abrasion. Lubricants are usually added shortly before pressing, as they should be present on the surface of the granules and between them and the parts of the press mold. The amount of the lubricant in the composition may vary between 0.05 and 15 % per weight, preferred between 0.2 and 5 % per weight, more preferred between 0.3 and 3 % per weight, most preferred between 0.3 and 1.5 % per weight.

Suitable lubricants are a.o. sodium oleate, metal stearates such as sodium stearate, calcium stearate, potassium stearate and magnesium stearate, stearic acid, sodium benzoate, sodium acetate, sodium chloride, boric acid, waxes having a high melting point, polyethylene glycol.

Emulsifiers can be selected for example from the following anionic and non-ionic emulsifiers: Anionic emulsifier waxes, cetyl alcohol, cetylstearyl alcohol, stearic acid, oleic acid, polyoxyethylene polyoxypropylene block polymers, addition products of 2 to 60 mol ethylene oxide to castor oil and/or hardened castor oil, wool wax oil (lanolin), sorbitan esters, polyoxyethylene alkyl esters, polyoxyethylene sorbitan fatty acid esters, polyoxyethene sorbitan monolaurate, polyoxyethene sorbitan monooleate, polyoxyethene sorbitan monopalmitate, polyoxyethene sorbitan monostearate, polyoxyethene sorbitan tristearate, polyoxyethene stearate, polyvinyl alcohol, metatartaric acid, calcium tartrate, alginic acid, sodium alginate, potassium alginate, ammonium alginate, calcium alginate, propane-1,2-diol alginate, carrageenan, processed eucheuma seaweed, locust bean gum, tragacanth, acacia gum, karaya gum, gellan gum, gum ghatti, glucomannane, pectin, amidated pectin, ammonium phosphatides, brominated vegetable oil, sucrose acetate isobutyrate, glycerol esters of wood rosins, disodium phosphate, trisodium diphosphate, tetrasodium diphosphate, dicalcium diphosphate, calcium dihydrogen diphosphate, sodium triphosphate, pentapotassium triphosphate, sodium polyphosphates, sodium calcium polyphosphate, calcium polyphosphates, ammonium polyphosphate, beta-cyclodextrin, powdered cellulose, methyl cellulose, ethyl cellulose, hydroxypropyl cellulose, hydroxypropyl methylcellulose, ethyl methyl cellulose, carboxymethyl cellulose, sodium carboxymethyl cellulose, ethyl hydroxyethyl cellulose, crosscarmellose, enzymically hydrolyzed carboxymethyl cellulose, mono- and diglycerides of fatty acids, glyceryl monostearate, glyceryl distearate, acetic acid esters of mono- and diglycerides of fatty acids, lactic acid esters of mono- and diglycerides of fatty acids, citric acid esters of mono- and diglycerides of fatty acids, tartaric acid esters of mono- and diglycerides of fatty acids, mono- and diacetyl tartaric acid esters of mono- and diglycerides of fatty acids, mixed acetic and tartaric acid esters of mono- and diglycerides of fatty acids, succinylated monoglycerides, sucrose esters of fatty acids, sucroglycerides, polyglycerol esters of fatty acids, polyglycerol polyricinoleate, propane-1,2-diol esters of fatty acids, propylene glycol esters of fatty acids, lactylated fatty acid esters of glycerol and propane-1, thermally oxidized soy bean oil interacted with mono- and diglycerides of fatty acids, dioctyl sodium sulphosuccinate, sodium stearoyl-2-lactylate, calcium stearoyl-2-lactylate, stearyl tartrate, stearyl citrate, sodium stearoyl fumarate, calcium stearoyl fumarate, stearyl tartrate, stearyl citrate, sodium stearoyl fumarate, calcium stearoyl fumarate, sodium laurylsulfate, ethoxylated mono- and diglycerides, methyl glucoside-coconut oil ester, sorbitan monostearate, sorbitan tristrearate, sorbitan monolaurate, sorbitan monooleate, sorbitan monopalmitate, sorbitan trioleate, calcium sodium polyphosphate, calcium polyphosphate, ammonium polyphosphate, cholic acid, choline salts, distarch glycerol, starch sodium octenyl succinate, acetylated oxidized starch.

Preferred are glycerin monooleate, stearic acid, phospholipids such as lecithin.

Stabilizers are substances that can be added to prevent unwanted changes in other supplements. Though stabilizers are not real emulsifiers they may also contribute to the stability of emulsions. Suitable examples for stabilizers are oxystearin, xanthan gum, agar, oat gum, guar gum, tara gum, polyoxyethene stearate, aspartame-acesulfame salt, amylase, proteases, papain, bromelain, ficin, invertase, polydextrose, polyvinyl pyrrolidone, polyvinyl polypyrrolidone, triethyl citrate, maltitol, maltitol syrup.

Diluents or fillers are inactive substances added to drugs in order to handle minimal amounts of active agents. Examples for suitable diluents are water, mannitol, pre-gelatinized starch, starch, microcrystalline cellulose, powdered cellulose, silicified microcrystalline cellulose, dibasic calcium phosphate dihydrate, calcium phosphate, calcium carbonate, hydroxypropyl cellulose, hydroxyethyl cellulose, hydroxypropyl methylcellulose, polyethylene glycol, xanthum gum, gum arabic or any combination thereof.

Anti-caking agents (antiadherents) can be added to a supplement or a composition of supplements in order to prevent the formation of lumps and for easing packaging, transport, release from the at least one chamber of the dispensing cap and consumption. Suitable examples include tricalcium phosphate, powdered cellulose, magnesium stearate, sodium bicarbonate, sodium ferrocyanide, potassium ferrocyanide, calcium ferrocyanide, bone phosphate, sodium silicate, silicon dioxide, calcium silicate, magnesium trisilicate, talcum powder, sodium aluminosilicate, potassium aluminium silicate, calcium aluminosilicate, bentonite, aluminium silicate, stearic acid, polydimethyl siloxane.

Sorbents are materials that soak up oil from the water. Suitable examples include natural sorbents such as peat moss, sawdust, feathers, and anything else natural that contains carbon and synthetic sorbents such as polyethylene and nylon. Sorbents are used for tablet/capsule moisture-proofing by limited fluid sorbing (taking up of a liquid or a gas either by adsorption or by adsorption) in a dry state.

Opacifiers are substances that render the drinkable liquid opaque, if desired. They must have a refractive index substantially different from the solvent, in most cases here water. At the same time they should be inert to the other components of the composition. Suitable examples include titanium dioxide, talc, calcium carbonate behenic acid, cetyl alcohol, or mixtures thereof.

All of the aforementioned substances and classes of substances can be used as supplements according to the invention, alone or in any conceivable combination thereof.

For the production of a dosage form as a 5-amino-2,3-dihydro-1,4-phthalazinedione containing suppository waxes with a low melting point as well as a mixture of fatty acid glycerides such as cocoa butter are first melted, then 5-amino-2,3-dihydro-1,4-phthalazinedione is homogenously dispersed under stirring or other mixing methods. The molten homogeneous mixture is then transferred to suitable molds and cooled down until solidification.

5-amino-2,3-dihydro-1,4-phthalazinedione can be also combined at least one active agent selected from a group comprising steroidal and non-steroidal anti-inflammatory drugs; immunomodulators; immunostimulatory agents; immunosuppressive agents; antibiotics; anti-infective agents; antiviral agents; antifungal agents; antiprotozoal agents; anthelmintics; analgesics; local anesthetics; anticoagulants; antiplatelet drugs; muscle relaxants; tonic agents; and anabolic agents for use in the prophylaxis and/or treatment of inflammatory and/or degenerative disorders of the tendons, ligaments of the joints, articular capsules and bursae.

Suitable examples for such steroidal anti-inflammatory drugs comprise corticosteroids, glucocorticoids, cortisone, cortisone acetate, hydrocortisone, hydrocortisone acetate, dexamethasone, betamethasone, prednisone, prednisolone, methylprednisolone, deltasone, triamcinolone, tixocortol pivalate, mometasone, amcinonide, budesonide, desonide, fluociconide, fluocinolone, halcinonide, fluocortolone, hydrocortisone-17-valerate, halometasone, alclometasone dipropionate, betamethasone valerate, betamethasone dipropionate, prednicarbate, clobetasone-17-butyrate, clobetasol-17-propionate, fluocortolone caproate, fluocortolone pivalate, fluprednidene acetate, hydrocortisone-17-butyrate, hydrocortisone-17-aceponate, hydrocortisone-17-buteprate, ciclesonide, flunisolide, fluticasone furoate, fluticasone propionate, triamcinolone acetonide, beclomethasone dipropionate.

Suitable examples for such non-steroidal anti-inflammatory drugs (NSAIDs) comprise acetylsalicylic acid, salicylic acid and salicylates, acetaminophen (paracetamol), salsalate, diflunisal, ibuprofen, dexibuprofen, naproxen, fenoprofen, ketoprofen, dexketoprofen, flurbiprofen, oxaprozin, loxoprofen, indomethacin, tolmetin, sulindac, etodolac, ketorolac, diclofenac, aceclofenac, nabumetone, piroxicam, meloxicam, tenoxicam, droxicam, lornoxicam, isoxicam, phenylbutazone, mefenamic acid, meclofenamic acid, flufenamic acid, tolfenamic acid, celexoxib, rofecoxib, valdecoxib, parecoxib, lumiracoxib, etoricoxib, firocoxib, nimesulide, clonixin, licofelone, H-harpagide, flunixin, tiaprofenic acid.

Suitable examples for such immunomodulators (IMIDs) comprise thalidomide, lenalidomide, pomalidomide and apremilast.

Suitable examples for such antiviral drugs comprise ancriviroc, aplaviroc, cenicriviroc, enfuvirtide, maraviroc, vicriviroc, amantadine, rimantadine, pleconaril, idoxuridine, aciclovir, brivudine, famciclovir, penciclovir, sorivudine, valaciclovir, cidofovir, ganciclovir, valganciclovir, sofosbusvir, foscarnet, ribavirine, taribavirine, filibuvir, nesbuvir, tegobuvir, fosdevirine, favipiravir, merimepodib, asunaprevir, balapiravir, boceprivir, ciluprevir, danoprevir, daclatasvir, narlaprevir, telaprevir, simeprevir, vanipevir, rupintrivir, fomivirsen, amenamevir, alisporivir, bevirimate, letermovir, laninamavir, oseltamivir, peramivir, zanamivir.

Suitable examples for such immunostimulatory agents comprise interferons (α-, β-, γ-, τ-interferon), interleukins, CSF, PDGF, EGF, IGF, THF, levamisole, dimepranole, inosine.

Suitable examples for such immunosuppressive drugs comprise the groups of glucocorticoids such as listed above; cytostatics such as alkylating agents (such as cyclophosphamide), antimetabolites such as methotrexate, azathioprine, mercaptopurine, fluorouracil, leflunomid, protein synthesis inhibitors and certain antibiotics such as dactinomycin, anthracyclines, mitomycin C, bleomycin and mithramycin, intercalating agents such as mitoxantrone; antibodies such as muromonab-CD3, rituximab, ustekinumab, alemtuzumab, natalizumab, basiliximab and daclizumab; drugs acting on immunophilins such as ciclosporin, tacrolimus and sirolimus; and non-classified immunosuppressive drugs such as β-interferon, γ--interferon, opioids, TNF binding proteins such as infliximab, etanercept, adalimumab; or curcumin, catechins, mycophenolic acid, fingolimod, myriocin and fumaric acid dimethyl esters.

Suitable examples for such antibiotics comprise imipenem, meropenem, ertapenem, cephalosporins, aztreonam, penicillins such as penicillin G and penicillin V, piperacillin, mezlocillin, ampicillin, amoxicillin, flucloxacillin, methicillin, oxacillin, clavulanic acid, sulbactam, tazobactam, sultamicillin, fosfomycin, teicoplanin, vancomycin, bacitracin, colistin, gramicidin, polymyxin B, tyrothricin, teixobactin, fosmidomycin, amikacin, gentamicin, kanamycin, neomycin, netilmicin, streptomycin, tobramycin, chloramphenicol, fusidic acid, cethromycin, narbomycin, telithromycin, clindamycin, lincomycin, daptomycin, dalfopristin, quinupristin, azithromycin, clarithromycin, erythromycin, roxithromycin, linezolid, doxycycline, minocycline, tetracycline, oxytetracycline, tigecycline, norfloxacin, enoxacin, ciprofloxacin, ofloxacin, levofloxacin, moxifloxacin, metronidazole, tinidazole, aminocumarine, sulfadiazine, sulfadoxin, sulfamethoxazole, sulfasalazine, pyrimethamine, trimethoprim, rifampicin.

Anti-infective agents is a generic term for compounds that are useful in the treatment of bacterial, viral, fungal, protozoal and worm infections and comprises antibiotics, antiviral agents, antimycotics agents and anthelminthic agents.

Suitable examples for such muscle relaxants comprise tercuronium, 1-ethylcarbamoyl-3-(3-trifluoromethylphenyl)pyrrolidine, metaxalone, methocarbamol, meprobamate, baclofen, carisoprodol, chlorzoxanzone, cyclobenzaprine, dantrolene, diazepam, orphenadrine, quinine, rocuronium, succinylcholine, decamethonium, pancuronium, veruronium, rapacuronium, dacuronium, duador, malouetine, dipyrandium, pipercuronium, chandonium, HS-342, atracurium, mivacurium, doxacurium, d-tubocurarine, dimethyltubocurarine, gallamine, alcuronium, anatruxonium, diadonium, fazadinium, tropeinium, cisatrucurium.

Suitable examples for such antifungal drugs comprise abafungin, amphotericin B, candicidin, filipin, hamycin, natamycin, nystatin, rimocidin, bifonazole, butoconazole, clotrimazole, econazole, fenticonazole, isoconazole, ketoconazole, luliconazole, miconazole, omoconazole, oxiconazole, sertaconazole, sulconazole, tioconazole, albaconazole, efinaconazole, epoxiconazole, fluconazole, isavuconazole, itraconazole, posaconazole, propiconazole, ravuconazole, terconazole, voriconazole, amorolfin, butenafine, nafitifine, terbinafine, anidulafungin, caspofungin, micafungin, benzoic acid, ciclopirox, flucytosine, griseofulvin, haloprogin, tolnaftate, undecylenic acid, crystal violet, balsam of Peru.

Suitable examples for such antiprotozoal drugs comprise metronidazole, tinidazole, ornidazole, atovaquone, clioquinol, chlorquinaldol, emetin, pentamidine isethionate, eflornithine, nitrofural, halofuginone, miltefosine, chloroquine, hydroxychloroquine, mepacrine, primaquine, amodiaquine, pamaquine, piperaquine, proguanil, cyclohunailembonate, quinine, mefloquine, pyrimethamine, artmether, artemisinine, artesunate, dihydroartemisinine, halofantrine, lumefantrine, sulfadoxine.

Suitable examples for such anthelmintics comprise mebendazole, praziquantel, albendazole, diethylcarbamazine, flubendazole, ivermectin, levamisole, metrifonate, niclosamide, oxyclozanide, oxamniquine, oxantel, piperazine, pyrantel, pyrantel pamoate, monopantel, derquantel, pelletierine sulphate, pyrvinium, thiabendazole, fenbendazole, triclabendazole, abamectin, suramine, emodepside, pyrvinium embonate, aminoacetonitrile.

Suitable examples for such local anesthetics comprise lidocaine, lignocaine, menthol, articaine, bupivacaine, ropivacaine, benzocaine, chloroprocaine, cocaine, cyclomethycaine, dimetociane, larocaine, piperocaine, propoxycaine, procaine, novocaine, proparacaine, tetracaine, amethocaine, cinchocaine, dibucaine, etidocaine, levobupivacaine, meplavacaine, prilocaine, trimecaine, saxitoxin, neosaxitoxin, tetrodotoxin, eugenol.

Suitable examples for analgesics comprise the NSAIDs listed above; opioid analgesics such as morphine, fentanyl, methadone, oxycodon, carfetanyl, dihydroetorphin, ohmefentanyl, etorphin, sufentanil, remifentanil, alfentanil, buprenorphine, hydromorphone, levomethadone, hydrocodone, pintramide, nalbuphine, tapentadol, pentazocin, dihydrocodeine, codeine, pethidine, tramadol, tilidine, meptazinol, naloxone, naltrexone, diprenorphine, loperamide, apomorphine; epibatidine; scopolamine; ziconitide; cannabinoids such as tetrahydrocannabinol, cannabidiol, marinol; flupirtine; ketamine and local anesthetics listed above.

Suitable examples for such anticoagulants comprise heparins, cumarins such as phenprocoumon (marcumar) and warfarin, apixaban, rivaroxaban, edoxaban, dabigatran, ximelagatran, hirudine, lepirudine, bivalirudine, citrate, EDTA, fondaparinux, argatroban, otamixaban.

Suitable examples for such antiplatelet agents comprise abciximab, acetylsalicylic acid, dipyridamole, clopidogrel, eptifibatide, ilomedin, prostacyclin, prasugrel, ticagrelor, ticlopidine, tirofiban.

Tonic agents is a generic term that refers to substances that invigorate, tone or restore the body and its physiological functions. They may be of herbal or animal origin.

Anabolic drugs may be useful for the anabolism and strengthening of the cellular collagen scaffold. However, there has been a broad abuse of these substances for doping in sports and for body building. Therefore the combinational use with 5-amino-2,3-dihydro-1,4-phthalazinedione is only encouraged as so far no national legal bans of these anabolic compounds are infracted.

According to the invention 5-amino-2,3-dihydro-1,4-phthalazinedione, a composition containing 5-amino-2,3-dihydro-1,4-phthalazinedione or a composition containing 5-amino-2,3-dihydro-1,4-phthalazinedione and at least one of the aforementioned combinational drugs for use in the prophylaxis and/or treatment of inflammatory and/or degenerative disorders of the tendons, ligaments of the joints, articular capsules and bursae can be applied orally, parenterally, intravenously, intraarterially, intramuscularly, topically, transdermally, subcutaneously, intradermally, sublingually, intravaginally, rectally or nasally.

As laid out before a considerable number of tendons, ligaments of the joints, articular capsules and bursae are poorly perfused. Therefore a systemic application is not always yielding the desired results, or increased dosages have to be administered. Therefore an alternative route of administration may be desirable in these cases. It was found that an injection of a 5-amino-2,3-dihydro-1,4-phthalazinedione compound, a composition containing said compound or of a composition containing said compound in combination with at least one of the drugs mentioned above into the affected soft tissue, the surrounding tissue or body fluid may lead to improved results in respect of drug efficacy and/or pharmacokinetics. Therefore the present application refers also to the use of said substance, composition or combination in the prophylaxis and/or treatment of inflammatory and/or degenerative disorders of the tendons, ligaments of the joints, articular capsules and bursae, wherein a parenteral application is carried out by injection of said substance, composition or combination into the affected tendon, ligament, articular capsule or bursa or into the surrounding tissue or body fluid, respectively.

Particularly good results, also in respect of patient compliance, are achieved when a jet injector as known in the art is used for such a parenteral injection.

If the affected tendon, ligament of the joints, articular capsule or bursae is located close to the skin surface it may be preferable to use a topical application for the same reasons as laid out before. Therefore the present application refers also to the use of said substance, composition or combination in the prophylaxis and/or treatment of inflammatory and/or degenerative disorders of the tendons, ligaments of the joints, articular capsules and bursae, wherein a topical application is carried out by means of a cream, emulsion, lotion, gel, hydrogel, paste, powder, ointment, liniment, film, liposomes, dermal patch, transdermal patch, transdermal spray or suspension.

Improved results in such topical applications in respect of efficacy and/or pharmacokinetics can be achieved when a permeation enhancer is included in the topical dosage form. Suitable permeation enhancers have been listed above.

The present patent application also refers to a method of treatment by using a 5-amino-2,3-dihydro-1,4-phthalazinedione compound for the prophylaxis and/or treatment of inflammatory and/or degenerative disorders of the tendons, ligaments of the joints, articular capsules and bursae in a patient in need thereof. The method comprises the following steps:
Providing a substance of the invention, a composition of the invention or a combination of the invention;
   and
administering said substance, composition or combination in an effective amount to said patient.

The terms "medicine" and "medical" comprise human as well as veterinary medicine.

According to the application the terms "active substance", "active principle" or "active pharmaceutical ingredient" (API) refer to the substances or the compounds of 5-amino-2,3-dihydro-1,4-phthalazinedione, as defined above.

The terms "composition" or "pharmaceutical composition" comprise at least one active principle in any pharmacologically acceptable defined dosage and dosage form together with at least one pharmaceutically acceptable excipient, as well as all agents that are generated from the aforementioned ingredients directly or indirectly as a combination, accumulation, complex or crystal, or as a consequence of other reactions or interactions, as well as optionally at least one further pharmaceutical drug, as listed above.

The term "excipient" is used in this application to describe any component of a pharmaceutical composition apart of the active principle. The selection of suitable excipients depends on a variety of factors, such as the dosage form, the dosage, the desired solubility and stability of the composition.

The term "effect" describes a specific mode of action intrinsic to the active principle in the scope of the present invention.

The terms "effect", "therapeutic effect", "action", "therapeutic action" in regard to the substance of the invention refers to causally occurring beneficial consequences in the organism to which said substance has been administered before.

According to the invention the terms "effective amount" and "therapeutically effective amount" refer to an amount of the substance of the invention that is sufficiently large to cause a desired beneficial effect in a person in need of such a prophylaxis or treatment.

The terms "prophylaxis", "treatment" and "therapy" comprise the administration of at least the substance of the invention, alone or in combination with at least one further pharmaceutical drug, independently of the chronological order of the administration. Such an administration is intended to prevent or inhibit the outbreak of one of the disorders of the invention, and /or to mitigate the symptoms and/or to initiate a healing process of such a disorder.

### Examples

### Example 1: Treatment of posterior tibial tendon dysfunction (PTTD)

In October 2012 an at this time 52 year old male, a physician and a recreational long-distance runner, went on a 10 km race, after a 6 weeks' training period in which he ran at a faster pace than usual.

In the first week of November he began suffering from an intense pain in the right foot that progressively worsened. After a further 10 days he wasn't able to run anymore. He stopped running but the pain persisted.

On visiting a podiatrist he was diagnosed with posterior tibial tendon dysfunction (PTTD). The treatment included applying a soft cast for immobilizing the affected foot and the medication of the NSAID Celebrex^{®} (Celecoxib) 100 mg twice a day for two weeks. Thereupon the acute pain diminished but the walking pain persisted.

After this period the podiatrist prescribed him insoles for arch support. They helped to relieve the pain and the affected foot became functional again for daily activities. However, night pain (a distinctive indicator of underlying continuous inflammation) persisted. It became so intense that the patient sometimes wasn't able to sleep. Also upon fast walking the pain persisted.

In February 2013, he attended a rheumatologist who found a severe tendinitis. In MRI a striking thinning and degenerative "shredding" of the affected tendon could be visualized. A further intake of Celebrex^{®} over one month diminished the acute pain again, but the walking pain and the night pain persisted.

In May to mid-June 2013 the patient underwent a shockwave therapy during a 6 weeks' course. Also this therapy was able to suppress the acute pain, but the night pain persisted.

The next therapeutic attempt included two injections of platelet-enriched plasma into the affected foot area. The first shot was in July 2013, the second in September 2013. Only a mild pain relief during the day could be achieved.

In November 2013 the patient attended an orthopedic surgeon who after thorough examination and judging by the MRI predicted him that he will never be able to run again. Physical therapy would be able to reach a stabilization of foot function, but not more.

In December 2013 he started to take one 25 mg tablet of 5-amino-2,3-dihydro-1,4-phthalazinedione sodium salt thrice a day over four weeks. After one week of this therapy the aforementioned symptoms began to vanish. After four weeks the pain, including the night pain, was completely gone and the patient was able to walk longer distances without complaints or restrictions.

Surprisingly, a further MRI revealed a significant strengthening of the affected tendon tissue.

In spring 2014 he resumed running. With a gradual increase in distance he was able to run up to 5 km at a reasonable pace by summer.

No resting pain or night pain of the affected foot has occurred since then.

### Example 2: Treatment of rotator cuff syndrome

In February 2015 the same patient as in Example 1 suffered a rotator cuff incident. During a heavy snowfall season he had repeatedly swept the snow from the eaves of his house by means of a roof rake he used to hold overhead for this purpose. One day he felt a strong pain in his right shoulder when doing so. The pain quickly progressed. Also after stopping this activity a severe resting and night pain developed that progressively worsened. Being a physician, he diagnosed himself with a rotator cuff syndrome that affected the whole right shoulder and its mobility. This included an injury of the supraspinatus tendon. In general it comes along with a bursitis of the subacromial bursa. Also an inflammation of the articular capsule of the acromio-clavicular joint (adhesive capsulitis of the shoulder) was obvious because of the acute poignant pain which is a typical sign when the densely innervated outer fibrous layer of the articular capsule is affected.

This time he started the other day to take 100 mg 5-amino-2,3-dihydro-1,4-phthalazinedione sodium salt twice a day over a period of ten days. After three to four days an improvement of the pain and the mobility of the shoulder started. After two weeks no impairment of the mobility was given anymore. The pain had subsided completely. No relapse has occurred since then.

### Example 3: Treatment of sacroiliitis

In March 2015 a 56 year old female started to suffer from persistent low back pain on the right side, radiating into the right buttock and thigh. The pain was described as rather dull and became worse during physical activities such as long walks. By way of diagnosis by exclusion the patient was finally diagnosed with right sided sacroiliitis. This disease is commonly due to an inflammation of the ligaments of the sacroiliac joint (anterior sacroiliac ligament, interosseous sacroiliac ligament, posterior sacroiliac ligament, sacrotuberous ligament, sacrospinous ligament). The anterior sacroiliac ligament is often regarded as an extension of the anterior joint capsule which is also affected by the inflammatory process.

Ibuprofen (800 mg, 3 times a day) was prescribed. Ibuprofen helped to reduce the pain, though not completely. During physical activity the pain was still present. Upon intents to withdraw the medication or at least to reduce the daily dosage after several weeks the pain flared up again as before the treatment. Thus only the pain was suppressed but no healing of the underlying inflammation could be observed.

Additional physiotherapy had only weak effects on the patient's well-being.

In August 2015 the patient stopped the intake of ibuprofen and started to take 100 mg tablets of 5-amino-2,3-dihydro-1,4-phthalazinedione sodium salt four times a day. While during the first three days the patient didn't observe any effect starting from day 4 a continuous reduction of the pain was sensed. After 10 days the same level of pain reduction was observed as with ibuprofen. After 21 days the patient felt no pain at all, neither under physical stress. The mobility restrictions due to pain had disappeared. She could also resume extended walks without any flaring up of pain. A reduction to three tablets a day, however, led to a slight worsening. No unwanted side effects were observed.

The observation of a continuous improvement is in favor of an anti-inflammatory action of 5-amino-2,3-dihydro-1,4-phthalazinedione sodium salt instead of a pure pain suppression which takes places rather fast.

### Example 4: Treatment of Tenosynovitis

In August 2015 a 32 year old male started suffering from tenosynovitis of the left thumb (De Quervain's tenosynovitis). The inflammation was probably due to overstrain during professional sports. Symptoms included pain over the wrist at the base of the thumb and a corresponding painful mobility impairment. The pain became worse during activity and was dampened after rest. The physician recommended a conservative therapy, including much resting of the left hand. During a four weeks' time no significant improvement of the symptoms could be observed. Thereafter the patient started to take 5-amino-2,3-dihydro-1,4-phthalazinedione sodium salt tablets four times a day (100 mg in total). While during the first 10 - 11 days no marked effect could be observed on day 11 the inflammatory pain started to fade away quite abruptly and was completely gone after one or two more days. Since then the pain didn't flare up again and the wrist, respectively the thumb has become fully operational again. The patient could resume his professional sport activities.

## Claims

1. 5-Amino-2,3-dihydro-1,4-phthalazinedione, its complexes, solvates, hydrates, crystalline polymorphs, tautomers, isomers, isotopically enriched forms, prodrugs or their pharmaceutically acceptable salts, for use in the prophylaxis and/or treatment of inflammatory and/or degenerative disorders of the tendons, ligaments of the joints, articular capsules and bursae.

2. The substance for use according to claim 1, wherein said substance is a crystalline polymorph of 5-Amino-2,3,dihydro-1,4-phthalazinedione sodium salt selected from a group comprising Form I, Form II and Form III,
wherein said Form I, Form II and Form III, respectively, are **characterized by** the following crystallography values determined by means of x-ray powder diagrams:
Form I:
d values: 13.5; 6.9; 5.2; 4.6; 3.9; 3.5; 3.4; 3.3; 3.1; 3.0 and/or
2-theta values: 6.5; 12.7; 16.9; 19.3; 22.8; 25.8; 26.6; 27.2; 28.7; 30.3;
Form II:
d values: 12.9; 7.9; 7.1; 6.5; 5.3; 4.0; 3.7; 3.6; 3.3; 3.2 and/or
2-theta values: 6.8; 11.2; 12.5; 13.7; 16.7; 22.4; 24.3; 24.9; 27.2; 27.8;
Form III:
d values: 13.131; 7.987; 7.186; 6.566; 6.512; 5.372; 3.994; 3.662; 3.406; 3.288; 3.283; 3.222; 3.215; 3.127; 2.889 and/or
2-theta values: 6.73; 11.07; 12.31; 13.48; 13.59; 16.49; 22.24; 24.29; 26.14; 27.10; 27.14; 27.67; 27.72; 28.52; 30.93.

3. The substance for use according to claim 1 or 2, wherein said of inflammatory and/or degenerative disorders of the tendons, ligaments of the joints, articular capsules and bursae becomes manifest in poorly perfused tissues.

4. The substance for use according to any of claims 1, 2 or 3, wherein said inflammatory and/or degenerative disorder of the tendons is selected from a group comprising tendinopathy; tendopathy; tendinitis; tenosynovitis; infective tenosynovitis; tendinosis; abscess of tendon sheath; calcific tendinitis; trigger finger; radial styloid tenosynovitis; tendovaginitis stenosans; contracture of the tendon or the tendon sheath; tenosynovitis as a symptom of gonorrhea, syphilis or tuberculosis; chronic crepitant tenosynovitis of hand and wrist; tendinitis trochanterica; rotator cuff tendinopathy; inflammations, lesions or tears of the rotator cuff tendons; bicipital tendinitis; calcific tendinitis of shoulder; supraspinatus syndrome; gluteal tendinitis; psoas tendinitis; enthesopathy; iliotibial band syndrome; patellar tendinitis; Achilles tendinopathy; Achilles tendinitis; peroneal tendinitis; tibialis anterior tendinitis; tibialis posterior tendinitis; posterior tibial tendon dysfunction (PTTD); medial epicondylitis; lateral epicondylitis and repetitive strain injury syndrome (RSI).

5. The substance for use according to any of claims 1, 2 or 3, wherein said inflammatory and/or degenerative disorder of the ligaments of the joints is selected from a group comprising ligamentopathy; ligamentitis; stenosing ligamentitis; sacroiliitis; irritations, stretches and tears of the ligaments; and ligamentous laxity.

6. The substance for use according to any of claims 1, 2 or 3, wherein said inflammatory and/or degenerative disorder of the articular capsules is selected from a group comprising synoviopathy; synovitis; infective synovitis; rupture of synovium; synovial hypertrophy; transient synovitis; synovial cysts; Baker's cyst; capsulitis; adhesive capsulitis of shoulder; adhesive capsulitis of the hip; and synovitis as a symptom of gonorrhea, syphilis or tuberculosis as well as of an autoimmune disease.

7. The substance for use according to any of claims 1, 2 or 3, wherein said inflammatory and/or degenerative disorder of bursae is selected from a group comprising bursopathy; bursitis; infective bursitis; bursitis of hand; Olecranon bursitis; bursitis of elbow; prepatellar bursitis; infrapatellar bursitis; bursitis of knee; trochanteric bursitis; bursitis of hip; subacromial bursitis; Achilles bursitis; retrocalcaneal bursitis; ischial bursitis; iliopsoas bursitis; anserine bursitis; abscess of bursa; bursal cyst; bursitis of shoulder; calcific bursitis; and bursitis as a symptom of gonorrhea, syphilis or tuberculosis as well as of an autoimmune disease.

8. A composition for use in the prophylaxis and/or treatment of inflammatory and/or degenerative disorders of the tendons, ligaments of the joints, articular capsules and bursae, wherein said composition contains at least one substance according to claim 1 or 2, a carrier and at least one excipient selected from a group comprising penetration enhancers; binding agents; antiadherents; solvents; solubilizing agents; coatings; colorants; disintegrants; flavoring substances; aromatic substances; thickening agents; glidants; lubricants; preservatives; sorbents; sweeteners; vehicles; foaming agents; emulsifiers; diluents; anti-caking agents; and opacifiers.

9. Combination of a substance according to claim 1 or 2 and at least one active agent selected from a group comprising steroidal and non-steroidal anti-inflammatory drugs; immunomodulators; immunostimulatory agents; immunosuppressive agents; antibiotics; anti-infective agents; antiviral agents; antifungal agents; antiprotozoal agents; anthelmintics; analgesics; local anesthetics; anticoagulants; antiplatelet drugs; muscle relaxants; tonic agents; and anabolic agents for use in the prophylaxis and/or treatment of inflammatory and/or degenerative disorders of the tendons, ligaments of the joints, articular capsules and bursae.

10. The substance of claim 1 or 2, the composition of claim 8 or the combination of claim 9 for use in the prophylaxis and/or treatment of inflammatory and/or degenerative disorders of the tendons, ligaments of the joints, articular capsules and bursae, wherein said substance, composition or combination is applied orally, parenterally, intravenously, intraarterially, intramuscularly, topically, transdermally, subcutaneously, intradermally, sublingually, intravaginally, rectally or nasally.

11. The substance of claim 1 or 2, the composition of claim 8 or the combination of claim 9 for use in the prophylaxis and/or treatment of inflammatory and/or degenerative disorders of the tendons, ligaments of the joints, articular capsules and bursae according to claim 10, wherein a parenteral application is carried out by injection of said substance, composition or combination into the affected tendon, ligament, articular capsule or bursa or into the surrounding tissue or body fluid, respectively.

12. The substance of claim 1 or 2, the composition of claim 8 or the combination of claim 9 for use in the prophylaxis and/or treatment of inflammatory and/or degenerative disorders of the tendons, ligaments of the joints, articular capsules and bursae according to claim 11, wherein a jet injector is used for said injection.

13. The substance of claim 1 or 2, the composition of claim 8 or the combination of claim 9 for use in the prophylaxis and/or treatment of inflammatory and/or degenerative disorders of the tendons, ligaments of the joints, articular capsules and bursae according to claim 10, wherein a topical application is carried out by means of a cream, lotion, gel, hydrogel, paste, powder, ointment, liniment, film, liposomes, dermal patch, transdermal patch, transdermal spray or suspension.

14. The substance of claim 1 or 2, the composition of claim 8 or the combination of claim 9 for use in the prophylaxis and/or treatment of inflammatory and/or degenerative disorders of the tendons, ligaments of the joints, articular capsules and bursae according to claim 13, wherein said topical application includes the use of a permeation enhancer.

15. A method for the prophylaxis and/or treatment of inflammatory and/or degenerative disorders of the tendons, ligaments of the joints, articular capsules and bursae in a patient in need thereof, the method comprising:
Providing a substance of claim 1 or 2, a composition of claim 8 or a combination of claim 9; and
administering said substance, composition or combination in an effective amount to said patient.
